# EUROPEAN PATENT SPECIFICATION

(11) **EP 3 384 041 B1**
(45) Date of publication and mention of the grant of the patent: **20.01.2021**
(21) Application number: 16805061.5
(22) Date of filing: 29.11.2016
(51) Int. Cl.: C12Q 1/37, G01N 33/543, G01N 33/58

(54) **METHOD FOR IDENTIFICATION OF PROTEASE SUBSTRATES**
VERFAHREN ZUR IDENTIFIKATION VON PROTEASESUBSTRATEN
PROCÉDÉ D'IDENTIFICATION DE SUBSTRAT DE PROTÉASE

(30) Priority: 30.11.2015 US 201562261191 P
(43) Date of publication of application: 10.10.2018
(73) Proprietor: F. Hoffmann-La Roche AG, 4070 Basel (CH); Roche Diagnostics GmbH, 68305 Mannheim (DE)
(72) Inventor: LYAMICHEV, Victor, Madison, WI 53711 (US); PATEL, Jigar, Verona, WI 53593 (US); SULLIVAN, Eric, Madison, WI 53716 (US); GOODRICH, Lauren, Madison, WI 53715 (US); KLEIN, Christian, 8906 Bonstetten (CH)
(74) Representative: HGF
(86) International application number: PCT/EP2016/079123
(87) International publication number: WO 2017/093246

(56) References cited:
- US-A1- 2010 093 554
- US-A1- 2015 185 216
- DEOK-HOON KONG ET AL: "Characterization of TAMRA- and biotin-conjugated peptide arrays for on-chip matrix metalloproteinase activity assay", BIOCHIP JOURNAL, vol. 6, no. 4, 20 December 2012 (2012-12-20), pages 307-313, XP055342385, SEOUL, SOUTH KOREA ISSN: 1976-0280, DOI: 10.1007/s13206-012-6401-3
- LÓPEZ-OTÍN C ET AL: "Protease degradomics: a new challenge for proteomics", NATURE REVIEWS MOLECULAR CELL BIOLOGY, MACMILLAN MAGAZINES, LONDON, GB, vol. 3, no. 7, July 2002 (2002-07), pages 509-519, XP002455899,
- SALISBURY C M ET AL: "Peptide microarrays for the determination of protease substrate specificity", JOURNAL OF THE AMERICAN CHEMICAL SOCIETY, AMERICAN CHEMICAL SOCIETY, US, vol. 124, no. 50, 18 December 2002 (2002-12-18), pages 14868-14870, XP002277823, ISSN: 0002-7863, DOI: 10.1021/JA027477Q
- DO-HYUN KIM ET AL: "Determination of protease subsite preference on SPOT peptide array by fluorescence quenching-based assay", JOURNAL OF PEPTIDE SCIENCE., vol. 18, no. 6, 30 June 2012 (2012-06-30), pages 394-399, XP055343963, GB ISSN: 1075-2617, DOI: 10.1002/psc.2409
- DATABASE Geneseq [Online] 22 April 2004 (2004-04-22), "TANGO 197-related thrombin cleavage peptide.", XP002766910, retrieved from EBI accession no. GSP:ADI00569 Database accession no. ADI00569 -& US 2003/144193 A1 (ROTTMAN JAMES B [US] ET AL) 31 July 2003 (2003-07-31)
- DATABASE Geneseq [Online] 9 December 2010 (2010-12-09), "Helical protein secondary structure fragment, SEQ ID 7709.", XP002766911, retrieved from EBI accession no. GSP:AYK82495 Database accession no. AYK82495
- DUAN Y J ET AL: "Protease Substrate Specificity Mapping Using Membrane-Bound Peptides", ANALYTICAL BIOCHEMISTRY, ELSEVIER, AMSTERDAM, NL, vol. 216, no. 2, February 1994 (1994-02), pages 431-438, XP024763379, ISSN: 0003-2697, DOI: 10.1006/ABIO.1994.1064 [retrieved on 1994-02-01]
- DIAMOND ET AL: "Methods for mapping protease specificity", CURRENT OPINION IN CHEMICAL BIOLOGY, CURRENT BIOLOGY LTD, LONDON, GB, vol. 11, no. 1, 9 February 2007 (2007-02-09), pages 46-51, XP005881385, ISSN: 1367-5931, DOI: 10.1016/J.CBPA.2006.11.021
- DOEZ R H P: "Profilig primary protease specificity by peptide synthesis on a solid support", ANGEWANDTE CHEMIE, WILEY - V C H VERLAG GMBH & CO. KGAA, DE, vol. 116, 14 June 2004 (2004-06-14), pages 3200-3203, XP002307314, ISSN: 0044-8249, DOI: 10.1002/ANGE.200353367

## Description

### BACKGROUND

The invention relates to protease enzymes and more specifically, to discovery of protease substrates using peptide microarrays.

Post-translational modification (PTM) of proteins regulates various cellular processes including intra- and extra-cellular signal transduction, catabolism, and other enzyme functions. Examples of PTM include proteolysis, phosphorylation, and other chemical modifications. Because PTM is able to modify protein function more rapidly than transcription and translation, PTM plays a crucial role in biological response. For example, blood coagulation, complement activation, and cortical reaction of an oocyte are biological responses involving protein activation via proteolytic cleavage. About 2% of protein-coding sequences in complex genomes sequenced so far are estimated to encode proteases. Understanding substrate specificity of these enzymes offers clues to biological pathways. Furthermore, identifying the substrate (s) for a given protease enables the creation of highly-specific protease inhibitors, which may have potential applications in the clinic and elsewhere.

Protein kinases also play a role in many cellular processes. Protein kinases are a broad class of proteins targeting various amino acids for modification (e.g., tyrosine, histidine, and serine-threonine kinases). Identifying a peptide sequence targeted by the kinase opens the door to design substrate-mimicking compounds that could serve as clinically relevant, competitive, and possibly irreversible inhibitors of kinases.

Unlike other PTMs such as phosphorylation and glycosylation, proteolysis does not generate a unique chemical handle allowing for enrichment of modified proteins. That is, proteolysis is destructive as opposed to being constructive as in the case of other PTMs. Accordingly, approaches for the identification of proteases and their substrate repertoires has grown into a field of study termed 'degradomics'. Currently several methods exist to identify protease substrates. These include one- and two-dimensional electrophoresis as well as methods for N-terminal identification. Some drawbacks associated with these methods include the inability to screen large libraries of potential substrate candidates, low yield of chemical modifications necessary for detection, high background, and the occurrence of false positive results. Existing methods for identification of protease substrates are also hindered by bias towards highly abundant proteolysis products and by the need to rely upon mass-spectrometry matching.

Some methods aim to find protease substrates present in a natural source. For example, whole-cell lysates can be subjected to 2-D gel electrophoresis to create a spatial layout for protease recognition. *See* Bredemeyer, A. et al. (2004) A proteomic approach to the discovery of protease substrates, PNAS 101:11785. Other methods involve artificial substrates. *See* Harris, J. et al., (2000) Rapid and general profiling of protease specificity by using combinatorial fluorogenic substrate libraries, PNAS 97:7754. This method involves a cleavable library of peptides that are synthesized on solid support and released into solution to create a complex mixture of protease substrates. Protease substrates can also be identified *in vivo* by co-transfecting a population of host cells with a protease-expressing plasmid and a library of plasmids encoding candidate substrates capable of fluorescence upon cleavage. *See* Kostallas G., et al. (2011) Substrate Profiling of Tobacco Etch Virus Protease Using a Novel Fluorescence-Assisted Whole-Cell Assay. PLoS ONE 6(1): e16136. doi:10.1371/journal.pone.0016136. US 2010/093554 discloses a peptide array with fluorescently labelled peptides for use in protease cleavage assays. Other methods involve *in silico* search for a substrate, *see e.g.,* Barkan, D. et al., ((2010) Predicting protease substrates using sequence and structure features, Bioinformatics 26:1714) utilizing support vector machine (SVM) algorithm to identify the sequence and structural features of protease substrates. However, as described previously, the aforementioned methods for identification of protease substrates can be low-throughput, time-consuming, and generally inefficient.

Accordingly, there is a need for improved processes and systems for the identification and maturation of protease substrates.

### SUMMARY

The present invention overcomes the aforementioned drawbacks by providing a method for identification of a protease substrate. The invention is defined by the scope of the appended claims.

In accordance with a first aspect of the present invention, a method of identifying a substrate for a protease, the method comprising the steps of: a) contacting a protease to one of a first microarray and a second microarray, each of the first microarray and the second microarray having a plurality of features, the first microarray and the second microarray having the same plurality of features, each feature comprising one or more sequences including a unique candidate protease substrate, the one or more sequences being linked to a solid support and further linked to a reporter peptide; wherein: the at least one sequence has a formula: [R₁]-[L₁]-[Z₁]-[X₁]-[Z₂]-[L₂]; wherein R₁ is the reporter peptide, L₁ and L₂ are each a spacer, Z₁ and Z₂ are each independently selected from a peptide sequence having between 0 and 3 amino acids, and X₁ is a peptide having a defined sequence of between 5 and 15 amino acids; b) contacting a detectable element to each of the first microarray and the second microarray to allow binding of the detectable element to the reporter peptide in the at least one sequence in each of the features of the first microarray and the second microarray; c) detecting a first signal resulting from binding of the detectable element to the reporter peptide in the at least one sequence in each of the features of the first microarray and a second signal resulting from binding of the detectable element to the reporter peptide in the at least one sequence in each of the features of the second microarray; d) comparing the first signal resulting from binding to the first microarray and the second signal resulting from binding to the second microarray to identify a difference in the first signal and the second signal; and e) identifying at least one candidate protease substrate in the features identified in step d) as a substrate for the protease, wherein the reporter peptide is a peptide binder to a protein and the detectable element is the protein to which the peptide binder specifically binds.

In one embodiment, the reporter peptide is a peptide epitope and the detectable element is an antibody specific for the peptide epitope.

In yet another embodiment, the candidate protease substrate includes a core sequence selected from natural and non-natural amino acids.

In accordance with a second aspect of the present invention, a use of a peptide microarray for identifying a substrate for a protease according to the method of the first aspect, the peptide microarray comprising a plurality of features, each feature comprising one or more sequences including a unique candidate protease substrate, the one or more sequences being linked to a solid support and further linked to a reporter peptide, wherein the at least one sequence has the formula:

[R₁]-[L₁]-[Z₁]-[X₁]-[Z₂]-[L₂]

wherein: R₁ is a reporter peptide, L₁ and L₂ are each a spacer, Z₁ and Z₂ are independently selected from a peptide sequence having from 0 to 3 amino acids, and X₁ is a peptide having a defined sequence of from 5 to 15 amino acids.

In a further embodiment, the detectable element is streptavidin, and the peptide binder is selected from the group consisting of WTHPQFE, DYLAEYHGG, YERPGWKLS, PAPAWAHGG, NSFDEWLQK, WTHPQFEQK, ADYLAEYHGG, YERPGWKLGT, DPAPAWAHGG and NSFDDWLAKGG.

In one embodiment, the detectable element contains a fluorescent group.

The at least one sequence has the formula:

[R₁]-[L₁]-[Z₁]-[X₁]-[Z₂]-[L₂]

wherein R₁ is the reporter peptide, L₁ and L₂ are each a spacer, Z₁ and Z₂ are each independently selected from a peptide sequence having between 0 and about 3 amino acids, and X₁ is a peptide having a defined sequence of between about 5 and about 15 amino acids.

In still another embodiment, R₁ is a peptide sequence bound by streptavidin, at least one of L₁ and L₂ is a 6-hexanoic acid spacer, and the peptide sequence of Z₁ and Z₂ includes at least one of glycine and serine.

In one embodiment, L₂ is linked to the solid support.

In accordance with another embodiment of the present disclosure, a peptide substrate for the thrombin protease includes a sequence selected from the group consisting of SEQ ID NO:1 through SEQ ID NO:9.

In accordance with yet another embodiment of the present disclosure, a peptide substrate for the matriptase protease, the peptide substrate including a sequence selected from the group consisting of SEQ ID N0:10 through SEQ ID NO:28.

In accordance with still another embodiment of the present disclosure, a peptide microarray for identifying a substrate for a protease includes a plurality of features, each feature having at least one sequence linked to a solid support, the sequence including a candidate protease substrate peptide and a reporter peptide.

In one embodiment, the candidate protease substrate peptide and the reporter peptide are connected via a spacer.

In another embodiment, the spacer comprises an oleic acid.

In yet another embodiment, the spacer is a polymer of hexanoic acid.

The candidate protease substrate peptide comprises between 5 and 15 amino acids.

In still another embodiment, the reporter peptide is streptavidin binding sequence.

The at least one sequence has the formula:

[R₁]-[L₁]-[Z₁]-[X₁]-[Z₂]-[L₂]

wherein R₁ is the reporter peptide, L₁ and L₂ are each a spacer, Z₁ and Z₂ are each independently selected from a peptide sequence having between 0 and 3 amino acids, and X₁ is a peptide having a defined sequence of between 5 and 15 amino acids.

The present disclosure also provides a method of identifying a substrate for a protease includes the steps of: a) contacting a protease to a first array having a plurality of features, each feature including at least one sequence linked to a solid support, the at least one sequence including a candidate protease substrate linked to a detectable element capable of generating a detectable signal upon proteolytic digestion of the candidate protease substrate; b) detecting the signal resulting from proteolytic digestion of the candidate protease substrate in one or more features; c) identifying the candidate protease substrate in the features where signal has been detected in step b) as substrates for the protease.

The present disclosure further provides for a method of identifying a protease substrate using a peptide microarray. In one embodiment, the invention is a method of identifying a substrate for a protease comprising the steps of contacting the protease to a first solid support comprising multiple addressable features, each feature containing a candidate protease substrate peptide and a reporter peptide; contacting a detectable element to the first solid support to allow binding of the detectable element to the reporter peptides in the features of the first solid support; contacting the detectable element to the second solid support, which is identical to the first solid support but has not been contacted with the protease, to allow binding of the detectable element to the reporter peptides in the features of the second solid support; detecting the signal resulting from binding of the detectable element to the reporter peptides in the features of the first and the second solid support; comparing the signal resulting from binding to the first and the second solid support to identify the signal present in the second but not the first solid support; identifying the candidate protease substrate peptides in the missing features as substrates for the protease. The reporter peptide may be an epitope and the detectable element may be an antibody specific for that epitope. The reporter peptide may be a peptide binder to a protein and the detectable element is the protein to which the peptide binder specifically binds. The peptide binder may be selected by the method described in U.S. Application Ser. No. 14/577,334 filed on December 19, 2014. The detectable element may be streptavidin and streptavidin binders may be selected from the group consisting of WTHPQFE, DYLAEYHGG, YERPGWKLS, PAPAWAHGG, NSFDEWLQK, WTHPQFEQK, ADYLAEYHGG, YERPGWKLGT, DPAPAWAHGG and NSFDDWLAKGG. The detectable element may contain a fluorescent group.

The disclosure provides a microarray for identifying a substrate for a protease comprising addressable features, each feature containing a candidate protease substrate peptide and a reporter peptide. The candidate protease substrate peptide and the reporter peptide may be connected via a spacer, *e.g.,* a spacer comprising an oleic acid, *e.g.,* a polymer of hexanoic acid. The candidate protease substrate peptide may comprise between 5 and 15 amino acids. The reporter peptide may be streptavidin binding sequence.

The disclosure provides a substrate for a protease identified by a method comprising the steps of: contacting the protease to a first solid support comprising multiple addressable features, each feature containing a candidate protease substrate peptide and a reporter peptide; contacting a detectable element to the first solid support to allow binding of the detectable element to the reporter peptides in the features of the first solid support; contacting the detectable element to the reporter peptides in the features of the first and the second solid support; comparing the signal resulting from binding to the first and the second solid support to identify the signal present in the second but not the first solid support; identifying the candidate protease substrate peptides in the missing features as substrates for the protease.

The disclosure provides a method of identifying a substrate for a protease comprising the steps of: contacting the protease to a solid support comprising multiple addressable features, each feature containing a candidate protease substrate peptide and a detectable element capable of generating a detectable signal upon proteolytic digestion of the candidate protease substrate peptide; detecting the signal resulting from proteolytic digestion of the candidate protease substrate peptide in one or more features; identifying the candidate protease substrate peptides in the features where signal has been detected as substrates for the protease.

The disclosure provides a method of identifying substrates with at least two different affinities for a protease comprising the steps of: under a first set of conditions, contacting the protease to a solid support comprising multiple addressable features, each feature containing a candidate protease substrate peptide and a detectable element capable of generating a detectable signal upon proteolytic digestion of the candidate protease substrate peptide; detecting the signal resulting from proteolytic digestion of the candidate protease substrate peptide in one or more features under the first set of conditions; identifying the candidate protease substrate peptides in the features where signal has been detected in step b. as substrates for the protease with the first affinity; under a second set of conditions, contacting the protease to the same solid support as in the first step; detecting the signal resulting from proteolytic digestion of the candidate protease substrate peptide in one or more features under the second set of conditions; identifying the candidate protease substrate peptides as substrates for the protease with the second affinity.

The foregoing and other aspects and advantages of the invention will appear from the following description. In the description, reference is made to the accompanying drawings which form a part hereof, and in which there is shown by way of illustration a preferred embodiment of the invention. Such embodiment does not necessarily represent the full scope of the invention, however, and reference is made therefore to the claims and herein for interpreting the scope of the invention.

### BRIEF DESCRIPTION OF THE DRAWINGS

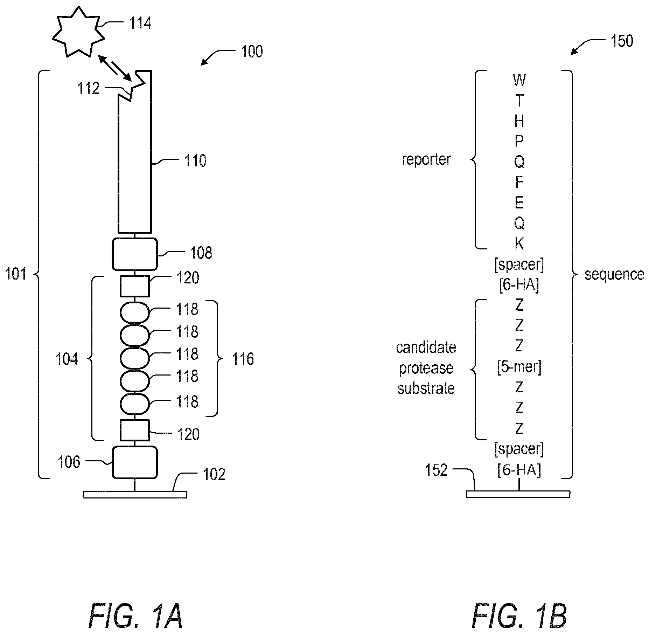

Figure 1A is an illustration of a single feature of a microarray for the identification of a protease substrate according to the present disclosure.
Figure 1B is an illustration of a particular example of a single feature of a microarray for the identification of a protease substrate according to the present disclosure. The features include a candidate protease substrate including a 5-mer core sequence and a reporter element exemplified as a streptavidin binder peptide.

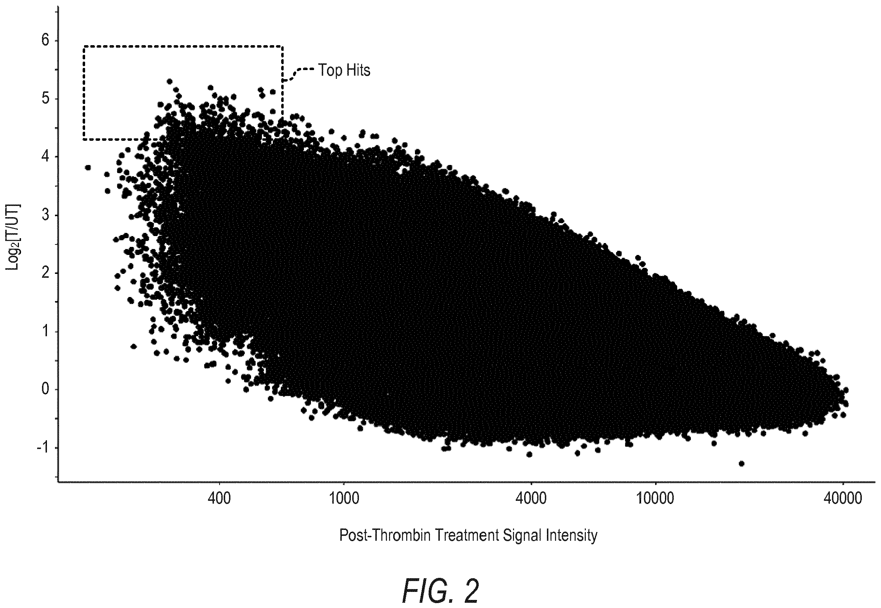

Figure 2 is a scatter plot showing results of cleavage of peptide features according to Figure 1B, with the protease Thrombin. The vertical axis shows the ratio of the fluorescence signal produced by Cy5-labeled streptavidin bound to feature for a peptide microarray treated (T) with thrombin relative the fluorescence signal produced by Cy5-labeled streptavidin bound to features from an untreated (UT) peptide microarray, while the horizontal axis shows the fluorescence signal for thrombin treated features only.

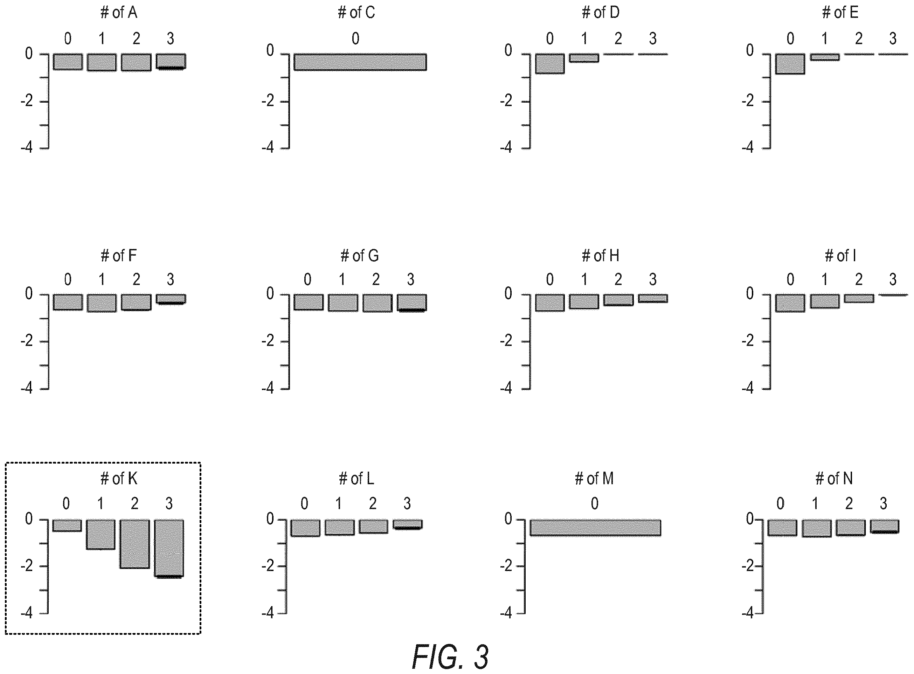

Figure 3 is a diagram showing the relationship between Thrombin cleavage efficiency and the number of a given amino acid in the 5-mer core sequence of the candidate substrate peptide. Data is shown for the amino acids alanine (A), cysteine (C), aspartic acid (D), glutamic acid (E), phenylalanine (F), glycine (G), histidine (H), isoleucine (I), lysine (K), leucine (L), methionine (M), and asparagine (N). For example, the first panel at the top left shows the log cleavage efficiency as a function of the number of alanine residues (0-3) in the 5-mer core sequence.

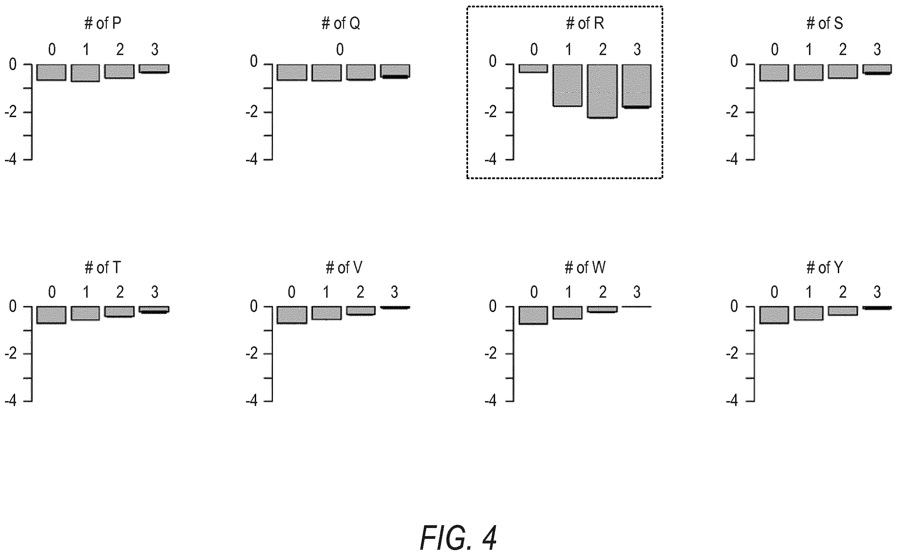

Figure 4 is a diagram showing the relationship between Thrombin cleavage efficiency and the number of a given amino acid in the 5-mer core sequence of the candidate substrate peptide. Data is shown for the amino acids proline (P), glutamine (Q), arginine (R), serine (S), threonine (T), valine (V), tryptophan (W), and tyrosine (Y). For example, the first panel at the top left shows the log cleavage efficiency as a function of the number of proline residues (0-3) in the 5-mer core sequence.

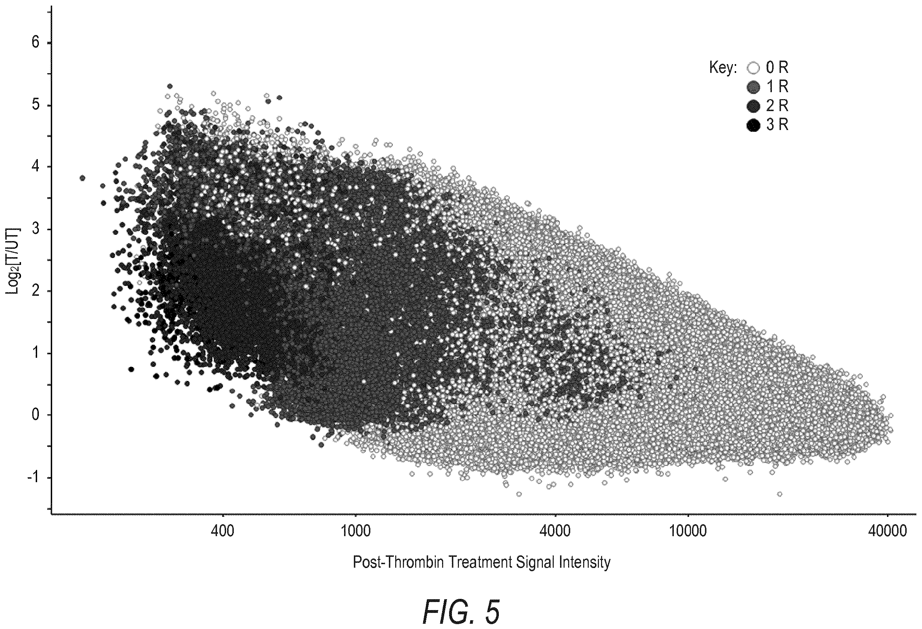

Figure 5 is the scatter plot of Figure 2 further illustrating results of cleavage of 5-mer peptides with Thrombin highlighting arginine (R) content (0-3 residues) in the peptide.

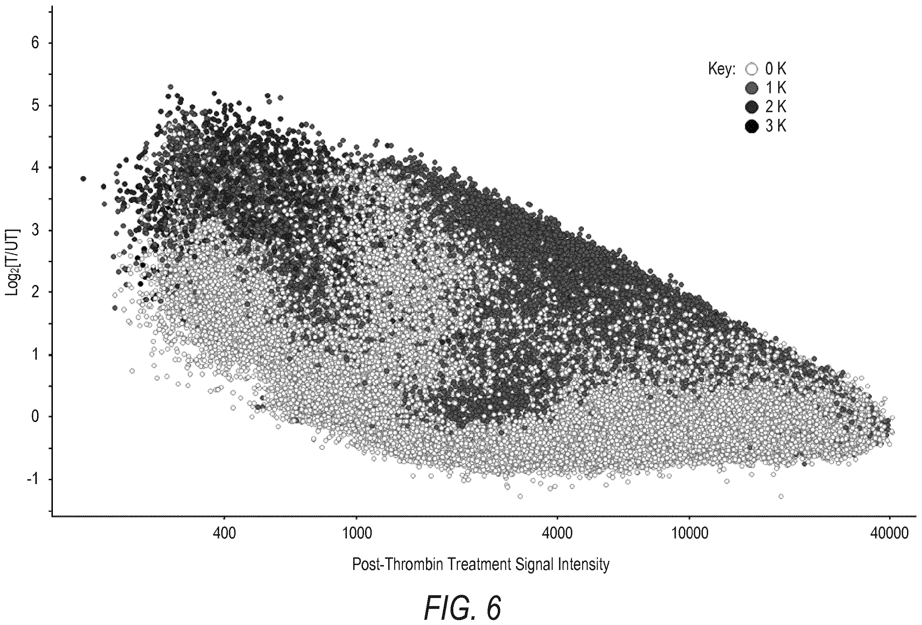

Figure 6 is the scatter plot of Figure 2 further showing results of cleavage of 5-mer peptides with Thrombin highlighting Lysine (K) content in the peptide.

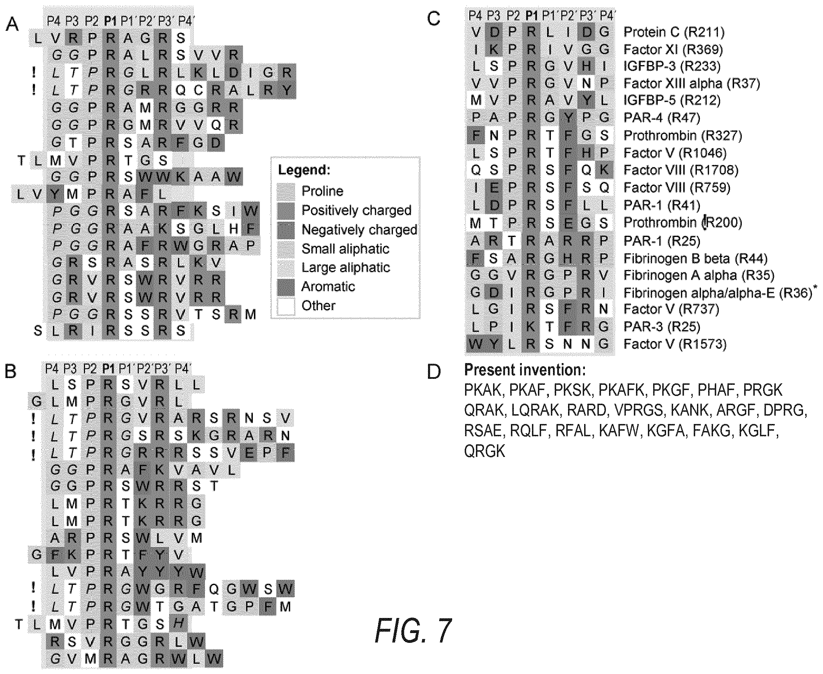

Figure 7 is a comparison of the lists of previously described and newly identified thrombin substrates according to the present disclosure. Panels A-C are from Gallwitz M., et al. (2012) The Extended Cleavage Specificity of Human Thrombin. PLoS ONE 7(2): e31756. Panel D is a list of peptide sequences identified through the methods of the present disclosure.

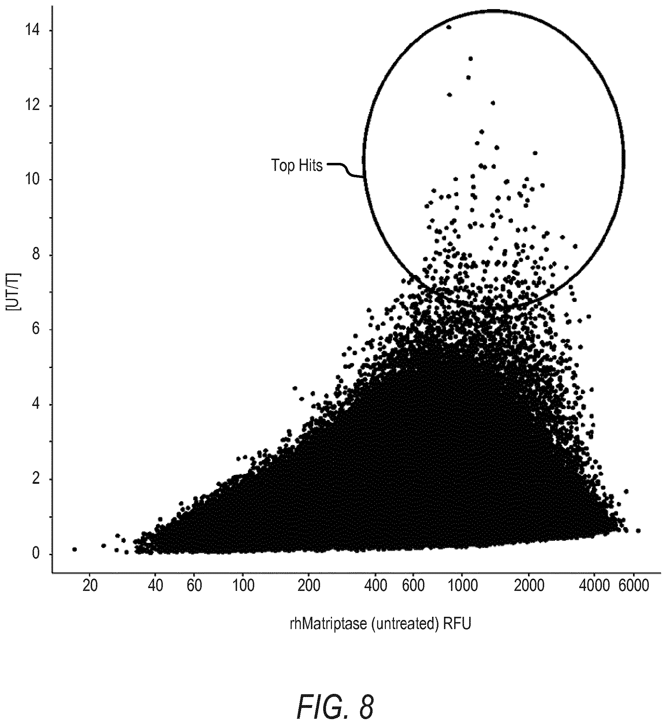

Figure 8 is a scatter plot showing results of cleavage of peptide features according to Figure 1B, with the protease matriptase. The vertical axis shows the ratio of the fluorescence signal produced by Cy5-labeled streptavidin bound to features on an untreated (UT) peptide microarray relative to the fluorescence signal produced by Cy5-labeled streptavidin bound to features on a peptide microarray treated with matriptase, while the horizontal axis shows the fluorescence signal for untreated features only.

### DETAILED DESCRIPTION

### I. Definitions

As used herein, the terms "peptide" and "oligopeptide" refer to organic compounds composed of amino acids, which may be arranged in either a linear or cyclic chain of amino acids joined together by peptide bonds between the carboxyl and amino groups of adjacent residues. The terms "peptide" and "oligopeptide" refer to shorter polypeptides, i.e., organic compounds composed of less than 50 amino acid residues.

The term "natural amino acid" refers to one of the 20 amino acids encoded by the standard genetic code and typically found in proteins and used for protein biosynthesis as well as other amino acids which can be incorporated into proteins during translation (including pyrrolysine and selenocysteine). The 20 natural amino acids include histidine, alanine, valine, glycine, leucine, isoleucine, aspartic acid, glutamic acid, serine, glutamine, asparagine, threonine, arginine, proline, phenylalanine, tyrosine, tryptophan, cysteine, methionine, and lysine.

The term "non-natural amino acid" refers to an organic compound that is not among those encoded by the standard genetic code, or incorporated into proteins during translation. The non-natural amino acids include amino acids or analogs of amino acids, for example, the D-isostereomers of amino acids (D-amino acids), the beta-amino-analogs of amino acids, citrulline, homocitrulline, homoarginine, hydroxyproline, homoproline, ornithine, 4-amino-phenylalanine, cyclohexylalanine, α-aminoisobutyric acid, N-methylalanine, N-methyl-glycine, norleucine, N-methyl-glutamic acid, tert-butylglycine, α-aminobutyric acid, tert-butylalanine, 2-aminoisobutyric acid, α-aminoisobutyric acid, 2-aminoindane-2-carboxylic acid, selenomethionine, dehydroalanine, lanthionine, γ-amino butyric acid, and derivatives thereof wherein the amine nitrogen has been mono- or di-alkylated.

The terms "microarray" or "peptide microarray," or simply "array" refer to a two dimensional arrangement of features (oligopeptides) on the surface of a solid or semi-solid support. A single microarray or, in some cases, multiple microarrays (e.g., 3, 4, 5, or more microarrays) can be located on one solid support. The size of the microarrays depends on the number of microarrays on one solid support. The higher the number of microarrays per solid support, the smaller the arrays have to be to fit on the solid support. The arrays can be designed in any shape, but preferably they are designed as squares or rectangle. The ready to use product is the oligopeptide microarray on the solid or semi-solid support referred to as a "microarray slide."

The term "feature" refers to a defined area on the surface of a microarray. The feature comprises biomolecules, such as in the context of the present invention, peptides. One feature can contain biomolecules with different properties, such as different sequences or orientations, as compared to other features. The size of a feature is determined by two factors: i) the number of features on an array (the higher the number of features on an array, the smaller is each single feature); and ii) the number of individually addressable aluminum mirror elements which are used for the irradiation of one feature. The higher the number of mirror elements used for the irradiation of one feature, the bigger is each single feature. The number of features on an array may be limited by the number of mirror elements (pixels) present in the micro mirror device. For example, the state of the art micro mirror device from Texas Instruments, Inc. currently contains 4.2 million mirror elements (pixels), thus the number of features within such exemplary microarray is therefore limited by this number. However, it should be understood that the micro mirror device from Texas Instruments, Inc. is provided only for exemplary purposes and higher density arrays are or will become available.

The term "solid or semi-solid support" refers to any solid material, having a surface area to which organic molecules can be attached through bond formation or absorbed through electronic or static interactions such as covalent bond or complex formation through a specific functional group. The support can be a combination of materials such as plastic on glass, carbon on glass, and the like. The functional surface can be simple organic molecules but can also comprise of co-polymers, dendrimers, molecular brushes, and the like.

As used herein, the term "spacer" refers to one or more chemical compounds, polymers or combinations thereof but does not include natural amino acids.

### II Description

The present invention relates to a method of screening a peptide library to identify enzyme substrate motifs. The enzyme is a protease. In variants of the disclosure, the enzyme is a protein kinase. The library is bound to a solid support i.e. a microarray. Methods of forming a peptide microarray are known in the art. Certain methods of producing peptide arrays comprise spotting prefabricated peptides or *in-situ* synthesis by spotting reagents on membranes (see U.S. Pat. No. 6,375,903). Other known methods used for generating peptide arrays of higher density involve photolithographic techniques, where the synthetic design of the desired biopolymers is controlled by suitable photolabile protecting groups (PLPG) releasing the linkage site for the respective next amino acid upon exposure to electromagnetic radiation, such as light (Fodor et al., (1993) Nature 364:555-556; Fodor et al., (1991) Science 251:767-773). Two different photolithographic techniques are known in the art. The first is a photolithographic mask, used to direct light to specific areas of the synthesis surface effecting localized deprotection of the PLPG. These "masked" methods include the synthesis of polymers utilizing a mount ("mask") which engages a substrate and provides a reactor space between the substrate and the mount. *See* U.S. Patent Nos. 5,143,854 and 5,445,934. The second photolithographic technique is the so-called maskless photolithography, where light is directed to specific areas of the synthesis surface effecting localized deprotection of the PLPG by digital projection technologies, such as micromirror devices (Singh-Gasson et al., Nature Biotechn. 17 (1999) 974-978). Such "maskless" array synthesis eliminates the need for time-consuming and expensive production of exposure masks. The peptide microarrays utilized in the method of the present invention may be synthesized by any of the methods described above or any other methods known in the art including the method previously described by the inventors in U.S. Application Ser. No. 14/577,334, entitled "Systemic Discovery, Maturation and Extension Of Peptide Binders to Proteins" filed on December 19, 2014.

### 1. Array Features

In some embodiments, the present disclosure includes the use of an array of peptide features disposed on a solid support. Each feature on the array has a defined position and sequence. Moreover, each feature can include a one or more identical sequences depending on factors such as the number of starting reactive sites within a given feature, the percent conversion of reactive sites to completed sequences, and the fidelity of the synthesis. For example, a hypothetical feature on a peptide array can encompass a 10 µm x 10 µm square at a defined coordinate on a solid support. The example feature can further have an estimated 10⁶ reactive sites per 10 µm² area, thereby enabling the synthesis of up to 10⁶ identical sequences within the feature. In another aspect, the sequence can include any number of different chemical building blocks, such as amino acids (e.g., natural and non-natural amino acids), linkers or spacers, fluorophores, the like, and combinations thereof.

Turning to Figure 1A, an example feature 100 includes a sequence 101 linked to a solid support 102. For simplicity, the peptide feature 100 is illustrated as including a single sequence 101 linked or bound to the solid support 102; however, it will be appreciated that the peptide feature 100 can be synthesized to include a plurality of identical sequences 101 within the area defined by the peptide feature 100 on the solid support 102. The sequence 101 includes a candidate protease substrate 104 that is linked to the solid support 102 via a first spacer 106. The substrate 104 is further linked via a second spacer 108 to a reporter 110. In the illustration shown in Figure 1A, the reporter 110 includes a binding portion 112 that is capable of interacting with and binding to a detectable element 114. For example, the reporter 110 can be a peptide sequence, the binding portion 112 can be a subset of amino acids in the peptide sequence representing a binding motif, and the detectable element 114 can be a fluorescently labeled antibody that specifically binds to the binding motif of the peptide sequence.

In the illustrated embodiment, the substrate 104 includes a core sequence 116 composed of individual monomer units 118. The core sequence 116 is further flanked by one or more additional monomer units 120. In one embodiment, the core sequence 116 is a 5-mer peptide having a defined amino acid sequence, and the core sequence 116 is flanked at each end with a 3-mer amino acid sequence (i.e., monomers 120) having a random or defined sequence. In summary, beginning from the end of the sequence 101 that is linked to the solid support 102, the sequence 101 includes the first spacer 106, one or more monomer units 120, the core sequence 116 composed of the monomer units 118, one or more monomer units 120, the second spacer 108, and the reporter 110 that is capable of interacting with the detectable element 114.

In one embodiment of the present disclosure, the feature 100 is useful for the identification of a protease substrate. For example, if the core sequence 116 is a substrate for a given protease, when the feature 100 is treated with the protease under conditions suitable for protease cleavage, the core sequence 116 will be cleaved, thereby separating the reporter 110 from the solid support 102 (e.g., the protease breaks the peptide bond between adjacent monomers 118 causing the reporter 110 to no longer be linked to or otherwise bound to the solid support 102). By contrast, if the core sequence 116 is not a substrate for the protease, when the feature 100 is treated with the protease, the core sequence will not be cleaved thereby leaving link between the reporter 110 and the solid support 102 (i.e., the core sequence 116) intact. Accordingly, following protease treatment, if the reporter 110 is detected at the defined location on the solid support 102 at which the feature 100 (and the sequence 101) was positioned, then it is likely that the core sequence 116 was not cleaved by the protease. However, if the reporter 110 is not detected at the location of the feature 100, then it is likely that the core sequence 116 was cleaved, and the core sequence 116 is therefore a likely substrate for the protease. These and other aspect of the present disclosure are described in greater detail herein, including in at least the Examples section below.

Turning now to Figure 1B, a particular example of a feature for identification of protease substrates is illustrated. As shown in Figure 1B, each feature 150 comprises one or more sequences including a unique candidate protease substrate linked to a solid support 152 and further linked to a reporter. In Figure 1B, the reporter is exemplified by a streptavidin-binding peptide sequence including the archetypical "HPQ" binding motif. However, the scope of the invention includes other peptide binding sequences. The scope of the invention also includes other reporters that are non-peptide based reporters. For example, the exemplified streptavidin-binding peptide sequence illustrated in Figure 1B can be replaced with a biotin molecule. The scope of the invention further includes embodiments where the reporter peptide is omitted and the detectable element is conjugated to the spacer or to the candidate peptide sequence itself. Yet other detection schemes are also included within the scope of the present disclosure.

Within the microarray feature 150, the candidate protease substrate is a peptide between about 5 and 15 amino acids long. In some embodiments, the candidate protease substrates are 5-mer peptides. Optionally, the peptides can be longer, *e.g.,* up to 11 amino acids long. In some embodiments, the peptides on the array are synthesized using only natural amino acids encoded by the standard genetic code. Non-natural amino acids may also be used as well as other molecules capable of forming peptide bonds. All 20 or fewer than 20, *e.g.,* only 18 natural amino acids can be used. In some embodiments, the array is synthesized using 18 natural amino acids and not including cysteine (Cys) and methionine (Met). In yet other embodiments, the peptide sequences on the array further exclude any dimer or a longer repeat of the same amino acid. In yet other embodiments, the peptide sequences on the array further exclude sequences known to have specificity for the test protease. The purpose of such exclusion is to avoid sequences already known to be substrates and encourage discovery of new substrates. This exclusion is especially advantageous when the test protease has very low dissociation constant (K_{D}) with the known substrates. By way of example, to avoid selecting for sequences HPQ and HPM, an array according to the method of the invention would exclude amino acid sequences HR, RH, HK, KH, RK, KR, HP, and PQ. Based on this example, one skilled in the art would be able to select sequences for exclusion from the array of candidates for a particular protease substrate.

In one example, a peptide array can have up to 2.9x10⁷ features, each feature having up to 10⁷ reactive sites that could yield a full length peptide. Smaller or larger arrays can also be designed. For example, an array representing a comprehensive list of all possible 5-mer peptides using all natural amino acids excluding cysteine will have 2,476,099 (∼2.5x10⁶) peptides. An array excluding certain amino acids and amino acid dimers can have about 1M (10⁶) peptides. In the case of maskless array synthesis (MAS), the number of feature on the array can correspond with the dimensions and number of total features of the digital micromirror device (DMD) used. In one example, a DMD having 10⁸ micromirrors can be used to prepare up to 10⁸ features.

### 2. Reporters

In the method of the present invention, the features on the microarray comprise a unique candidate protease substrate peptide conjugated or linked to a reporter peptide. In some embodiments, the reporter peptide is an amino acid sequence to which a detectable element specifically binds. Examples of a detectable element-reporter sequence pair include without limitation, antibody-epitope, and protein-peptide binder pair. An antibody against a specific epitope can be generated according to any method known in the art. Commercial antibodies against certain peptides are also available. Therefore any suitable epitope-antibody pair can be used as a reporter system in the context of the present invention.

In other embodiments, the reporter peptide is directly conjugated to a detectable element. In such embodiments, the reporter peptide serves a structural, sequence-independent role of containing the detectable element. In variants of the disclosure the reporter peptide may be omitted and the detectable element conjugated to the candidate substrate peptide either directly or via a linker. A detectable element directly conjugated to the peptide feature on the microarray may be a fluorescent molecule or any molecule capable of emitting a detectable signal. The signal may be either constant (e.g., a radioactive label, fluorescent reporter dye) or conditional (e.g., fluorescent donor dye transferring fluorescent energy to the later-added fluorescent acceptor dye that serves as a reporter dye).

In variants of the disclosure, the reporter peptide can be omitted and the fluorophore can be added directly to the N-terminus of the candidate protease substrate peptide. The loss of signal following protease digestion can be used to identify the candidate as the substrate for the test protease.

### 3. Protease-Resistant Reporters

In some embodiments, the reporter peptide has an additional property of protease resistance. In some instances, a test protease may unexpectedly have specificity for at least a part of the reporter peptide sequence thus thwarting the experimental design. To address such potential problems, the reporter peptide sequence may be designed to be resistant to proteolysis. For example, the reporter peptide sequence may contain amino acids normally not found in proteins (such as D-amino acids). In other embodiments, the amino acids may have chemical modification that would preclude proteolytic digestion of a peptide containing such modified amino acids. In general, any chemical modification that would prevent proteolysis of the reporter peptide sequence yet not interfere with the recognition by the detectable element or emission of a detectable signal by the detectable element is within the scope of the present invention. In variations of this embodiment, the reporter sequence is not a peptide sequence but an oligomer or a polymer that possesses necessary structural properties of the reporter sequence, i.e., support of the detectable element or specific recognition by the detectable element, but is resistant to protease digestion.

A peptide binder specifically binding to a target peptide may be identified by any method known in the art including the method previously described by the inventors in U.S. Application Ser. No. 14/577,334 *Systemic Discovery, Maturation and Extension Of Peptide Binders to Proteins* filed on December 19, 2014.

The reporter peptide sequence is connected to the candidate peptide sequence via a spacer (sometimes referred to as a linker). In some embodiments, the spacer is composed of one or more carboxylic acid molecules, for example, hexanoic acid. One of skill in the art would recognize similarities of chemical properties and interchangeability among the genus of carboxylic acids with aliphatic side chains to which hexanoic acid belongs. Furthermore, other compounds with similar chemical properties and steric characteristics to carboxylic acids with aliphatic side chains may be used in place of carboxylic acids. The linker is optional. In some embodiments, the candidate protease peptide and the reporter peptide may be connected directly, *e.g.,* via a peptide bond.

The reporter sequence is a peptide binder sequence that specifically binds to a protein suitable and convenient for use as a detectable element. For example, the reporter sequence may be a streptavidin-binding sequence with streptavidin being the detectable element. In some embodiments, the streptavidin-binding sequence is WTHPQFEQK. In other embodiments, other streptavidin-binding sequences are used, for example WTHPQFE, DYLAEYHGG, YERPGWKLS, PAPAWAHGG, NSFDEWLQK, WTHPQFEQK, ADYLAEYHGG, YERPGWKLGT, DPAPAWAHGG, or NSFDDWLAKGG. Longer or shorter versions or substituted versions of these sequences may also be used as long as they have sufficient affinity to streptavidin.

In other embodiments, the reporter sequence is a hexa-histidine sequence (6His) and the detectable element is a nickel(II) - nitrilotriacetic acid system (Ni²⁺-NTA) conjugated to a fluorophore, e.g., (Ni²⁺-NTA)₂-Cy3. *See* Zhao, C. et al. (2010). Hexahistidine-tag-specific optical probes for analyses of proteins and their interactions, Analytical Biochemistry 399 (2):237-45*.* In yet other embodiments, the reporter sequence is an epitope tag, *i.e.,* a reporter peptide sequence to which a high-affinity antibody is available for use as a detectable element. Some examples of epitope tags include Myc-tag (derived from c-Myc), HA-tag (derived from influenza hemagglutinin), and the artificial FLAG-tag (Hopp, T., et al. (1988) A Short Polypeptide Marker Sequence Useful for Recombinant Protein Identification and Purification, BioTechnology, 6 (10):1204).

### 4. Protease Substrate Candidates

As shown in Figure 1B, each peptide feature 150 comprises a candidate protease substrate peptide. The candidate protease substrate peptides within the feature may be 5 amino acids long or longer. In some embodiments, during array synthesis, the peptides may be extended on the N-terminus, C-terminus, or both termini by one or more amino acids. Such extension may be "wobble synthesis" wherein a mixture of 2 or more amino acids is used for incorporation. In some embodiments, the "wobble mixture" contains glycine (G) and serine (S) at a ratio of 3:1. Other examples the wobble mixture contains equal concentrations (e.g., equal ratios) of G, S, adenine (A), valine (V), aspartic acid (D), proline (P), glutamic acid (E), leucine (L), threonine (T) and/or equal concentrations (e.g., equal ratios) of amino acids L, A, D, lysine (K), T, glutamine (Q), P, F, V, tyrosine (Y). In this embodiment, the resulting protease substrate candidate peptides will have a combination of random and directed synthesis amino acids. For example, as shown in Figure 1, a candidate peptide on the array may be a 15-mer having the format: ZZZZZ - 5mer - ZZZZZ, where Z is an amino-acid from a particular wobble mixture.

### 5. Cleavage

The present invention is a method comprising a step of cleaving a protease substrate peptide among the population of unique candidate protease substrate peptides present on a solid support (*e.g.,* microarray). The method comprises a step of exposing the solid support with the unique candidate protease substrate peptides to the test protease under the conditions when the test protease is enzymatically active. Under such conditions, the substrate peptides among the candidates will be cleaved while the non-substrate peptides will remain intact on the solid support. One of skill in the art skill would appreciate that the same protease may have varying activity, *i.e.* different kinetic properties (K_{D}) with each substrate. The same protease may likewise have preferred reaction conditions under which the protease achieves maximum activity. The conditions are characterized by temperature, pH, and composition of the reaction buffer. Within the scope of the present invention is therefore a step of exposing the solid support with the unique candidate protease substrate peptides to the test protease under various reaction conditions to enable cleavage and identification of preferred and less preferred substrates for the protease.

The present invention is a method comprising a step of identifying which peptides among the population of unique candidate protease substrate peptides present on a solid support (*e.g.,* microarray) have been cleaved. In some embodiments, the method includes one or more wash steps that remove products of proteolytic cleavage. The identification of remaining (uncleaved) peptides utilizes a detectable element or another like detectable element. To that end, the method comprises a step of contacting the solid support previously exposed to the test protease with the detectable element. The detectable element will generate a detectable signal only within features where no proteolytic cleavage has occurred. Only with those features, the intact candidate peptide and the attached reporter sequence are still present on the solid support. In embodiments where the detectable element is directly conjugated to the candidate peptide on the microarray (*e.g.,* a fluorescent molecule or any molecule capable of emitting a detectable signal) the signal will likewise be detected only within features where no proteolytic cleavage has occurred.

The method further comprises a step of analyzing a second microarray identical to the first microarray and treated in identical manner, except not exposed to the test protease prior to being contacted with the detectable element. For example, the second microarray may be optionally exposed to the identical protease buffer solution not containing the protease and incubated under identical conditions as the first microarray. The candidate peptide sequences in the features detected on the second array but not on the first array are identified as substrates for the test protease.

### 6. Identification of Substrates

In the first aspect, the invention is a method of identifying a protease substrate using a peptide microarray. The method comprises a step of contacting the test protease with a first solid support (such as microarray) having addressable features, wherein each feature comprising a unique candidate protease substrate peptide. Each feature further comprises a reporter peptide sequence conjugated or linked to the candidate peptide sequence. In variants of the disclosure, each feature comprises a detectable element instead of the reporter peptide sequence to which a detectable element could bind.

The method further comprises a step of detecting the presence of uncleaved peptide sequences on the first solid support following exposure to the test protease by detecting the presence of the reporter peptide sequence on the first solid support. The step comprises contacting the first solid support with a detectable element under conditions allowing emission of the detectable signal either by binding to the reporter peptide sequence or by another mechanism leading to the signal being emitted only if the peptide within the feature has not been cleaved. The method further comprises a step of detecting the presence of peptide sequences on the second solid support that has not been treated with the protease. The method further comprises a step of comparing the detection results of the first and second solid support to identify the candidate protease substrate peptides present on the second but not on the first solid support. Such peptides have been cleaved by the protease off the first solid support and thus are identified as protease substrate peptides.

### 7. Array and System for Identifying Substrates

Variants of the disclosure provide a microarray for identifying a substrate for a protease. The microarray comprises addressable features, each feature comprises a unique candidate protease substrate peptide linked to the solid support and further linked to a reporter peptide sequence. Variants of the disclosure provide a system for identifying a substrate for a protease. The system comprises at least two microarrays, each microarray comprising addressable features, each feature containing a unique candidate protease substrate peptide linked to the solid support and further linked to a reporter peptide sequence or directly to a detectable element. The system may also comprise a separate detectable element. In some variants, the detectable element is detectable directly and in other embodiments, the detectable element is detectable indirectly, *e.g*., with a secondary antibody conjugated to a detectable substrate. In some variants, the system may further comprise detection means for detecting the presence of certain addressable features on a microarray. For example, if a detectable element comprises or is conjugated to a fluorescent group or fluorescent label, the label can be detected by a fluorescence scanner. Other labels and corresponding detection methods are chemiluminescence, colorimetry, or autoradiography.

In some variants the system further comprises computational means of comparing the population of features detected on each of the microarrays to identify the features present on one but missing from the other of the two microarrays. After scanning the microarray slides, the scanner records a 20-bit, 16-bit, or 8-bit numeric image that enables interpretation of each fluorescent spot on the scanned microarray slide. In some variants the result is qualitative, i.e., the detectable element is detected as present or absent corresponding to digestion or no digestion of the candidate peptide. The computational means are capable of correlation of the signal and the corresponding peptide sequence on the first microarray and the second microarray and identifying peptide sequences present on the second but not the first microarray. In some variants, the system further comprises reporting means for reporting the candidate peptide sequences as protease substrate sequences if they were detected on the untreated microarray but not detected on the protease-treated microarray.

### 8. Protease Substrates Identified According to the Present Disclosure

Variants of the disclosure provide a peptide substrate for a protease identified by a novel method described herein. The method comprises the steps of 1) contacting the protease with a first solid support (such as microarray) having addressable features, wherein each feature situated on the solid support comprises a candidate protease substrate peptide and a reporter peptide sequence conjugated or linked to the candidate peptide sequence; 2) contacting the first solid support with a detectable element to generate a detectable signal; and 3) detecting the presence of the reporter sequence on the first solid support; 4) repeating steps 2) and 3) with a solid support that has not been contacted with the protease; 5) identifying the features present on the second but not the first solid support thus identifying peptides in those features as substrates for the protease.

The peptide substrates described herein may be subjected to an *in vitro* evolution process (e.g., as described in U.S. Application Ser. No. 14/577,334) to obtain additional protease substrate peptides.

### 9. Confirmation of Substrates

In some embodiments, the method further comprises confirmation of identified candidates as substrates for the test protease. The confirmation may include a competitive or non-competitive proteolytic assay performed including the test protease and the candidate peptide with or without additional sequences and performed in solution or on solid support.

### 10. Specific Substrates

In some embodiments, the invention is a method of identifying a substrate for thrombin using a peptide microarray. Several natural and synthetic thrombin substrates have been described. Gallwitz M., et al. (2012) The Extended Cleavage Specificity of Human Thrombin. PLoS ONE 7(2): e31756. doi:10.1371/journal.pone.0031756. In the context of the present invention, the method comprises a step of contacting thrombin to a first solid microarray having addressable features, wherein each feature comprises a candidate thrombin substrate peptide. In some embodiments, each feature further comprises a reporter peptide sequence conjugated or linked to the candidate peptide sequence. The method further comprises a step of detecting the presence of the candidate peptide sequence by detecting the presence of the reporter sequence on the first microarray. The step comprises contacting the first microarray with a detectable element under conditions allowing its binding to the reporter sequence. After optional removal of the unbound detectable elements, the presence of the bound detectable elements is detected thereby detecting the presence of each candidate protease substrate peptide remaining on the solid support after treatment with thrombin. In some embodiments, the bound detectable element is directly detectable. In other embodiments, the detectable element is contacted with another molecule that is capable of emitting a detectable signal. The method further comprises a second microarray identical to the first microarray and treated in identical manner, except not exposed to thrombin prior to contacting and detecting the detectable element. The candidate peptide sequences in the features detected as present on the second array but not the first array are identified as substrates for the thrombin protease.

### Single Array Methods

Variants of the disclosure provide a method of identifying a protease substrate using a peptide microarray. The method comprises a step of contacting the test protease with a single solid support (such as microarray) having addressable features, wherein each feature situated on the solid support comprises a candidate protease substrate peptide directly conjugated to the detectable element capable of emitting a detectable signal upon cleavage with the protease. The detectable element may be *e.g.,* a fluorescent reporter dye paired with a fluorescence quencher prior to proteolytic digestion. Following proteolytic digestion of the peptide with the test protease, the quencher molecule is separated from the reporter fluorophore allowing fluorescence to occur. The candidate protease substrates in the features where cleavage has occurred and the fluorescent signal is detected are identified as protease substrate peptides.

In this variant, the same solid support (*e.g.,* microarray) can be subjected to sequential exposures to the test protease to identify substrates with varying affinities for the protease. Exposing the solid support to the protease under conditions less optimal for protease digestion enables identification of substrates with the highest affinity. After the high-affinity substrates have been removed from the solid support by proteolysis, exposing the solid support to the protease under a series of different, increasingly permissive, more optimal conditions enables identification of categories of substrates with decreasing affinity.

In some variants, the method comprises determining kinetic properties of candidate protease substrates by contacting the solid support containing candidate protease substrates with the protease and measuring the rate of proteolytic reaction over time where completion of the reaction is manifested by emission of a detectable signal.

### EXAMPLES

The following Examples are meant to be illustrative and are not intended to be limiting in any way.

### Example 1. Identifying candidate Thrombin binding sequences

Peptide microarrays were synthesized using maskless array synthesis according to the present disclosure. Each microarray contained features having the following structure in order from N-terminus to C-terminus:
WTHPQFEQK-[6-hexanoic acid]-[3Z]-[5-mer core sequence]-[3Z]-[6-hexanoic acid]-[solid support] (see Figure 1)
3Z represents a series of three "Z" amino acids where Z represents a mixture of one or more amino acids whose purpose is to convey solubility and
flexibility to the 5-mer core sequence. In one aspect the number of Z amino acids flanking the 5-mer core sequence can vary from 0 to 3 or more amino acids. In the present example, Z was a 3:1 mixture of Gly:Ser. However, in other examples, Z is a simply Glycine only (see Example 2). In another aspect, each 5-mer candidate peptide feature was synthesized with a pre-defined amino acid sequence, with each unique peptide feature having a different defined amino acid sequences. Accordingly, the amino acid sequence of the 5-mer candidate at each location on the microarray was known.

Two identical microarrays were used in the experiment. Following synthesis of the peptide features, one microarray was treated with thrombin protease (EC 3.4.21.5) under standard commercial conditions using a thrombin cleavage kit from EMD Millipore (Billerica, Mass.). Following thrombin treatment, both arrays were bound with streptavidin conjugated with the fluorescent dye Cy5. Both arrays were scanned on a fluorescence-based scanner platform to identify the fluorescence output for each feature on the array.

A comparison between the test and control arrays elicited top hits (Figure 2). In particular, data from treated and untreated arrays was analyzed by plotting the ratio of the fluorescence signal for each treated peptide feature relative to the fluorescence signal for the corresponding untreated peptide feature as a function of the fluorescence signal from the treated peptide features. Peptide features having low fluorescence signal following treatment but a large ratio of fluorescence signal for the treated and untreated features (e.g., the top left corner of Figure 2) were identified as top hits for further investigation (Table 1). The presence of each amino acid in the thrombin substrate peptides was also analyzed (Figures 3 and 4). The total number of arginine (R) or lysine (K) residues (0-3) within a given 5-mer core sequence exhibited the greatest correlation with thrombin cleavage. Moreover, visualization of the number of arginine or lysine residues for the data illustrated in Figure 2 revealed a unique clustering pattern for features having a similar number of either arginine or lysine residues as illustrated in Figures 5 and 6, respectively.

**Table 1:**

| **Thrombin Substrate Core Sequence** |
|---|
| PKAKX (SEQ ID NO:1) |
| PKSKX (SEQ ID NO:2) |
| PKAFK (SEQ ID NO:3) |
| QRAKX (SEQ ID NO:4) |
| RARDX (SEQ ID NO:5) |
| LQRAK (SEQ ID NO:6) |
| VPRGS (SEQ ID NO:7) |
| KANKX (SEQ ID NO:8) |
| QRGKX (SEQ ID NO:9) |

In Figure 7, the thrombin substrates identified by the method of the invention (panel D) were compared with previously reported thrombin substrates (panels A,-C from Gallwitz M., et al. (2012) The Extended Cleavage Specificity of Human Thrombin. PLoS ONE 7(2): e31756.)

### Example 2. Identifying candidate Matriptase binding sequences

Peptide microarrays were synthesized as in Example 1 with the goal of identifying substrates for the protease matriptase (EC 3.4.21.109). One notable difference is that Z was a glycine only solution (i.e., 3Z = GGG).

A first (treated) microarray slide was bound with 0.052µg/µl of rhMatriptase in Assay buffer (50 mM Tris, 50 mM NaCl, 0.01% (v/v) Tween® 20, pH 9.0), and a second microarray slide, was bound with only Assay buffer (i.e., untreated). Both microarrays were incubated overnight at room temperature.

Following overnight incubation, microarray slides were removed in 1X Tris Buffered Saline (TBS), washed for 30s in 1X TBS, and then washed for 30s in water.

For detection of cleavage, microarray slides were bound with streptavidin-Cy5 buffer: 640µl 1M Tris-Cl,pH 7.4, 6.4ml 5% Alkali-Soluble Casein, 16µl Tween20, 25.28 H2O, 150µl Streptavidin-Cy5 (1µg/µl). Microarray slides were incubated for 1 hr in the presence of the Cy5-labeled streptavidin, and then washed in 1X TBS for 30s, washed with water for 15s, and finally spun dry.

Scanning of the microarray slides was performed at 15%PMT Cy5 with a fluorescence scanner (Innopsys) at 1µm resolution.

Probe replicates were averaged and standard error (SE) was calculated for both arrays. A ratio of untreated to treated relative fluorescence units (RFU) was calculated, and the SE cutoff was set to 0.20.

A comparison between the test and control arrays elicited top hits (Figure 8). In particular, data from treated and untreated arrays was analyzed by plotting the ratio of the fluorescence signal for each untreated peptide feature relative to the fluorescence signal for the corresponding treated peptide feature as a function of the fluorescence signal from the untreated peptide features. Untreated peptide features having high fluorescence signal but a large ratio of fluorescence signal for the untreated and treated features (e.g., the top right corner of Figure 8) were identified as top hits for further investigation. The top hits included the matriptase substrate motifs listed in Table 2. Notably, sequences in Table 2 represent the core sequences of the overall candidate protease substrate shown in parenthesis for each table.

**Table 2:**

| **Matriptase Substrate Core Sequence** |
|---|
| AKSNS (SEQ ID NO:10) |
| EGKKN (SEQ ID NO:11) |
| ERQYK (SEQ ID NO:12) |
| GQAKN (SEQ ID NO:13) |
| HQAKG (SEQ ID NO:14) |
| IQARK (SEQ ID NO:15) |
| ISPKK (SEQ ID NO:16) |
| KKINH (SEQ ID NO:17) |
| KKLQT (SEQ ID NO:18) |
| LNARK (SEQ ID NO:19) |
| PSVKS (SEQ ID NO:20) |
| QESKK (SEQ ID NO:21) |
| QMAKK (SEQ ID NO:22) |
| QYKSS (SEQ ID NO:23) |
| RKANN (SEQ ID NO:24) |
| RNNQV (SEQ ID NO:25) |
| VNAKK (SEQ ID NO:26) |
| VQAKK (SEQ ID NO:27) |
| VQMFK (SEQ ID NO:28) |

## Claims

1. A method of identifying a substrate for a protease, the method comprising the steps of:
a. contacting a protease to one of a first microarray and a second microarray, each of the first microarray and the second microarray having a plurality of features, the first microarray and the second microarray having the same plurality of features, each feature comprising one or more sequences including a unique candidate protease substrate, the one or more sequences being linked to a solid support and further linked to a reporter peptide,
wherein:
the at least one sequence has a formula:
[R₁]-[L₁]-[Z₁]-[X₁]-[Z₂]-[L₂]
wherein:
R₁ is a reporter peptide,
L₁ and L₂ are each a spacer,
Z₁ and Z₂ are independently selected from a peptide sequence having between 0 to 3 amino acids, and
X₁ is a peptide having a defined sequence of from 5 to 15 amino acids;
b. contacting a detectable element to each of the first microarray and the second microarray to allow binding of the detectable element to the reporter peptide in the at least one sequence in each of the features of the first microarray and the second microarray;
c. detecting a first signal resulting from binding of the detectable element to the reporter peptide in the at least one sequence in each of the features of the first microarray and a second signal resulting from binding of the detectable element to the reporter peptide in the at least one sequence in each of the features of the second microarray;
d. comparing the first signal resulting from binding to the first microarray and the second signal resulting from binding to the second microarray to identify a difference in the first signal and the second signal;
e. identifying at least one candidate protease substrate in the features identified in step d as a substrate for the protease,
wherein the reporter peptide is a peptide binder to a protein and the detectable element is the protein to which the peptide binder specifically binds.

2. The method of claim 1, wherein the reporter peptide is a peptide epitope and the detectable element is an antibody specific for the peptide epitope.

3. The method of claim 1, wherein the-each of the first microarray and the second microarray comprises up to 2.9 x 10⁷ features and each feature has up to 10⁷ reactive sites.

4. The method of claim 1, wherein the candidate protease substrate includes a core sequence selected from natural and non-natural amino acids.

5. The method of claim 1, wherein the detectable element is streptavidin, and the peptide binder is selected from the group consisting of WTHPQFE, DYLAEYHGG, YERPGWKLS, PAPAWAHGG, NSFDEWLQK, WTHPQFEQK, ADYLAEYHGG, YERPGWKLGT, DPAPAWAHGG and NSFDDWLAKGG.

6. The method of claim 1, wherein the detectable element contains a fluorescent group.

7. Use of a peptide microarray for identifying a substrate for a protease according to the method of claim 1, the peptide microarray comprising a plurality of features, each feature comprising one or more sequences including a unique candidate protease substrate, the one or more sequences being linked to a solid support and further linked to a reporter peptide, wherein the at least one sequence has the formula:
[R₁]-[L₁]-[Z₁]-[X₁]-[Z₂]-[L₂]
wherein:
R₁ is a reporter peptide,
L₁ and L₂ are each a spacer,
Z₁ and Z₂ are independently selected from a peptide sequence having from 0 to 3 amino acids, and
X₁ is a peptide having a defined sequence of from 5 to 15 amino acids.

8. The use of claim 7, wherein the reporter peptide is streptavidin binding sequence.

9. The use of claim 7, wherein the peptide microarray comprises up to 2.9 x 10⁷ features and each feature has up to 10⁷ reactive sites.

## Patentansprüche

1. Verfahren zum Identifizieren eines Substrats für eine Protease, wobei das Verfahren die folgenden Schritte umfasst:
a. Inkontaktbringen einer Protease mit einem ersten Microarray oder einem zweiten Microarray, wobei das erste Microarray und das zweite Microarray jeweils eine Mehrzahl von Merkmalen aufweisen, wobei das erste Microarray und das zweite Microarray die gleiche Mehrzahl von Merkmalen aufweisen, wobei jedes Merkmal eine oder mehrere Sequenzen umfasst, einschließlich eines einzigartigen möglichen Proteasesubstrats, wobei die eine oder mehreren Sequenzen mit einem festen Träger und ferner mit einem Reporterpeptid verbunden sind,
wobei:
die mindestens eine Sequenz die folgende Formel aufweist:
[R₁]-[L₁]-[Z₁]-[X₁]-[Z₂]-[L₂]
wobei folgendes gilt:
R₁ ist ein Reporterpeptid;
L₁ und L₂ sind jeweils ein Spacer;
Z₁ und Z₂ sind unabhängig ausgewählt aus einer Peptidsequenz mit 0 bis 3 Aminosäuren; und
X₁ ist ein Peptid mit einer definierten Sequenz von 5 bis 15 Aminosäuren;
b. Inkontaktbringen eines nachweisbaren Elements mit jedes des ersten Microarrays und des zweiten Microarrays, um eine Bindung des nachweisbaren Elements an das Reporterpeptid in der mindestens einen Sequenz in jedem der Merkmale des ersten Microarrays und des zweiten Microarrays zu ermöglichen;
c. Nachweisen eines ersten Signals als Folge der Bindung des nachweisbaren Elements an das Reporterpeptid in der mindestens einen Sequenz in jedem der Merkmale des ersten Microarrays und eines zweiten Signals als Folge der Bindung des nachweisbaren Elements an das Reporterpeptid in der mindestens einen Sequenz in jedem der Merkmale des zweiten Microarrays;
d. Vergleichen des ersten Signals als Folge der Bindung an das erste Microarray und des zweiten Signals als Folge der Bindung an das zweite Microarray, um eine Differenz zwischen dem ersten Signal und dem zweiten Signal zu identifizieren;
e. Identifizieren mindestens eines möglichen Proteasesubstrats in den in Schritt d identifizierten Merkmalen als ein Substrat für die Protease;
wobei das Reporterpeptid ein Peptidbinder an ein Protein ist, und wobei das nachweisbare Element das Protein ist, an welches der Peptidbinder spezifisch bindet.

2. Verfahren nach Anspruch 1, wobei das Reporterpeptid ein Peptidepitop ist, und wobei das nachweisbare Element für das Peptidepitop antikörperspezifisch ist.

3. Verfahren nach Anspruch 1, wobei das erste Microarray und das zweite Microarray jeweils bis zu 2,9 x 10⁷ Merkmale umfassen, und wobei jedes Merkmal bis zu 10⁷ reaktionsfähige Stellen aufweist.

4. Verfahren nach Anspruch 1, wobei das mögliche Proteasesubstrat eine aus natürlichen und nicht natürlichen Aminosäuren ausgewählte Kernsequenz aufweist.

5. Verfahren nach Anspruch 1, wobei das nachweisbare Element Streptavidin ist, und wobei der Peptidbinder ausgewählt ist aus der Gruppe bestehend aus WTHPQFE, DYLAEYHGG, YERPGWKLS, PAPAWAHGG, NSFDEWLQK, WTHPQFEQK, ADYLAEYHGG, YERPGWKLGT, DPAPAWAHGG und NSFDDWLAKGG.

6. Verfahren nach Anspruch 1, wobei das nachweisbare Element eine fluoreszierende Gruppe enthält.

7. Verwendung eines Peptid-Microarrays zum Identifizieren eines Substrats für eine Protease gemäß dem Verfahren nach Anspruch 1, wobei das Peptid-Microarray eine Mehrzahl von Merkmalen aufweist, wobei jedes Merkmal eine oder mehrere Sequenzen umfasst, einschließlich eines eindeutigen möglichen Proteasesubstrats, wobei die eine oder mehreren Sequenzen mit einem festen Träger und ferner mit einem Reporterpeptid verbunden sind,
wobei:
die mindestens eine Sequenz die folgende Formel aufweist:
[R₁]-[L₁]-[Z₁]-[X₁]-[Z₂]-[L₂]
wobei folgendes gilt:
R₁ ist ein Reporterpeptid;
L₁ und L₂ sind jeweils ein Spacer;
Z₁ und Z₂ sind unabhängig ausgewählt aus einer Peptidsequenz mit 0 bis 3 Aminosäuren; und
X₁ ist ein Peptid mit einer definierten Sequenz von 5 bis 15 Aminosäuren.

8. Verwendung nach Anspruch 7, wobei das Reporterpeptid eine Streptavidin-Bindungssequenz ist.

9. Verwendung nach Anspruch 7, wobei das Peptid-Microarray bis zu 2,9 x 10⁷ Merkmale umfasst, und wobei jedes Merkmal bis zu 10⁷ reaktionsfähige Stellen aufweist.

## Revendications

1. Procédé d'identification d'un substrat pour une protéase, le procédé comprenant les étapes suivantes :
a. mise en contact d'une protéase avec l'un d'un premier microréseau et d'un second microréseau, chacun du premier microréseau et du second microréseau ayant une pluralité de caractéristiques, le premier microréseau et le second microréseau ayant la même pluralité de caractéristiques, chaque caractéristique comprenant au moins une séquence comprenant un substrat de protéase candidat unique, l'au moins une séquence étant liée à un support solide et en outre liée à un peptide rapporteur,
l'au moins une séquence ayant une formule :
[R₁]-[L₁]-[Z₁]-[X₁]-[Z₂]-[L₂]
où :
R₁ est un peptide rapporteur,
L₁ et L₂ sont chacun un espaceur,
Z₁ et Z₂ sont indépendamment choisis parmi une séquence peptidique ayant entre 0 et 3 acides aminés, et
X₁ est un peptide ayant une séquence définie de 5 à 15 acides aminés ;
b. mise en contact d'un élément détectable avec chacune des caractéristiques du premier microréseau et du second microréseau pour permettre la liaison de l'élément détectable au peptide rapporteur dans l'au moins une séquence de chacune des caractéristiques du premier microréseau et du second microréseau ;
c. détection d'un premier signal résultant de la liaison de l'élément détectable au peptide rapporteur dans l'au moins une séquence dans chacune des caractéristiques du premier microréseau et d'un second signal résultant de la liaison de l'élément détectable au peptide rapporteur dans l'au moins une séquence dans chacune des caractéristiques du second microréseau ;
d. comparaison du premier signal résultant de la liaison au premier microréseau et du second signal résultant de la liaison au second microréseau pour identifier une différence entre le premier signal et le second signal ;
e. identification d'au moins un substrat de protéase candidat parmi les caractéristiques identifiées à l'étape d comme substrat de la protéase,
le peptide rapporteur étant un liant peptidique à une protéine et l'élément détectable étant la protéine à laquelle le liant peptidique se lie spécifiquement.

2. Procédé selon la revendication 1, le peptide rapporteur étant un épitope peptidique et l'élément détectable étant un anticorps spécifique de l'épitope peptidique.

3. Procédé selon la revendication 1, chacun du premier microréseau et du second microréseau comprenant jusqu'à 2,9 x 10⁷ caractéristiques et chaque caractéristique ayant jusqu'à 10⁷ sites réactifs.

4. Procédé selon la revendication 1, le substrat de protéase candidat comprenant une séquence centrale choisie parmi les acides aminés naturels et non naturels.

5. Procédé selon la revendication 1, l'élément détectable étant la streptavidine, et le liant peptidique étant choisi dans le groupe constitué par WTHPQFE, DYLAEYHGG, YERPGWKLS, PAPAWAHGG, NSFDEWLQK, WTHPQFEQK, ADYLAEYHGG, YERPGWKLGT, DPAPAWAHGG et NSFDDWLAKGG.

6. Procédé selon la revendication 1, l'élément détectable contenant un groupe fluorescent.

7. Utilisation d'un microréseau peptidique pour identifier un substrat pour une protéase selon le procédé selon la revendication 1, le microréseau peptidique comprenant une pluralité de caractéristiques, chaque caractéristique comprenant au moins une séquence incluant un substrat de protéase candidat unique, l'au moins une séquence étant liée à un support solide et en outre liée à un peptide rapporteur, l'au moins une séquence ayant la formule :
[R₁]-[L₁]-[Z₁]-[X₁]-[Z₂]-[L₂]
où :
R₁ est un peptide rapporteur,
L₁ et L₂ sont chacun un espaceur,
Z₁ et Z₂ sont indépendamment choisis parmi une séquence peptidique ayant de 0 à 3 acides aminés, et
X₁ est un peptide ayant une séquence définie de 5 à 15 acides aminés.

8. Utilisation selon la revendication 7, le peptide rapporteur étant une séquence de liaison à la streptavidine.

9. Utilisation selon la revendication 7, le microréseau peptidique comprenant jusqu'à 2,9 x 10⁷ caractéristiques et chaque caractéristique ayant jusqu'à 10⁷ sites réactifs.


### SEQUENCE LISTING

<110> Roche Diagnostics GmbH F. Hoffmann-La Roche AG
<120> SYSTEM AND METHOD FOR IDENTIFICATION OF PROTEASE SUBSTRATES
<130> P33176-WO
<150> 62/261,191
   <151> 2015-11-30
<160> 28
<170> PatentIn version 3.5
<210> 1
   <211> 5
   <212> PRT
   <213> Artificial Sequence
<220>
   <223> Synthetic peptide sequence
<220>
   <221> 1
   <222> (5)..(5)
   <223> Any amino acid
<400> 1
   Pro Lys Ala Lys Xaa
   1               5
<210> 2
   <211> 5
   <212> PRT
   <213> Artificial Sequence
<220>
   <223> Synthetic peptide sequence
<220>
   <221> 1
   <222> (5)..(5)
   <223> any amino acid
<400> 2
   Pro Lys Ser Lys Xaa
   1               5
<210> 3
   <211> 5
   <212> PRT
   <213> Artificial Sequence
<220>
   <223> Synthetic peptide sequence
<400> 3
   Pro Lys Ala Phe Lys
   1               5
<210> 4
   <211> 5
   <212> PRT
   <213> Artificial Sequence
<220>
   <223> Synthetic peptide sequence
<220>
   <221> 1
   <222> (5)..(5)
   <223> any amino acid
<400> 4
   Gln Arg Ala Lys Xaa
   1               5
<210> 5
   <211> 5
   <212> PRT
   <213> Artificial Sequence
<220>
   <223> Synthetic peptide sequence
<220>
   <221> 1
   <222> (5)..(5)
   <223> any amino acid
<400> 5
   Arg Ala Arg Asp Xaa
   1               5
<210> 6
   <211> 5
   <212> PRT
   <213> Artificial Sequence
<220>
   <223> Synthetic peptide sequence
<400> 6
   Leu Gln Arg Ala Lys
   1               5
<210> 7
   <211> 5
   <212> PRT
   <213> Artificial Sequence
<220>
   <223> Synthetic peptide sequence
<400> 7
   Val Pro Arg Gly Ser
   1               5
<210> 8
   <211> 5
   <212> PRT
   <213> Artificial Sequence
<220>
   <223> Synthetic peptide sequence
<220>
   <221> 1
   <222> (5)..(5)
   <223> any amino acid
<400> 8
   Lys Ala Asn Lys Xaa
   1 5
<210> 9
   <211> 5
   <212> PRT
   <213> Artificial Sequence
<220>
   <223> Synthetic peptide sequence
<220>
   <221> 1
   <222> (5)..(5)
   <223> any amino acid
<400> 9
   Gln Arg Gly Lys Xaa
   1 5
<210> 10
   <211> 5
   <212> PRT
   <213> Artificial Sequence
<220>
   <223> Synthetic peptide sequence
<400> 10
   Ala Lys Ser Asn Ser
   1 5
<210> 11
   <211> 5
   <212> PRT
   <213> Artificial Sequence
<220>
   <223> Synthetic peptide sequence
<400> 11
   Glu Gly Lys Lys Asn
   1 5
<210> 12
   <211> 5
   <212> PRT
   <213> Artificial Sequence
<220>
   <223> Synthetic peptide sequence
<400> 12
   Glu Arg Gln Tyr Lys
   1 5
<210> 13
   <211> 5
   <212> PRT
   <213> Artificial Sequence
<220>
   <223> Synthetic peptide sequence
<400> 13
   Gly Gln Ala Lys Asn
   1 5
<210> 14
   <211> 5
   <212> PRT
   <213> Artificial Sequence
<220>
   <223> Synthetic peptide sequence
<400> 14
   His Gln Ala Lys Gly
   1 5
<210> 15
   <211> 5
   <212> PRT
   <213> Artificial Sequence
<220>
   <223> Synthetic peptide sequence
<400> 15
   Ile Gln Ala Arg Lys
   1 5
<210> 16
   <211> 5
   <212> PRT
   <213> Artificial Sequence
<220>
   <223> Synthetic peptide sequence
<400> 16
   Ile Ser Pro Lys Lys
   1 5
<210> 17
   <211> 5
   <212> PRT
   <213> Artificial Sequence
<220>
   <223> Synthetic peptide sequence
<400> 17
   Lys Lys Ile Asn His
   1 5
<210> 18
   <211> 5
   <212> PRT
   <213> Artificial Sequence
<220>
   <223> Synthetic peptide sequence
<400> 18
   Lys Lys Leu Gln Thr
   1 5
<210> 19
   <211> 5
   <212> PRT
   <213> Artificial Sequence
<220>
   <223> Synthetic peptide sequence
<400> 19
   Leu Asn Ala Arg Lys
   1 5
<210> 20
   <211> 5
   <212> PRT
   <213> Artificial Sequence
<220>
   <223> Synthetic peptide sequence
<400> 20
   Pro Ser Val Lys Ser
   1 5
<210> 21
   <211> 5
   <212> PRT
   <213> Artificial Sequence
<220>
   <223> Synthetic peptide sequence
<400> 21
   Gln Glu Ser Lys Lys
   1               5
<210> 22
   <211> 5
   <212> PRT
   <213> Artificial Sequence
<220>
   <223> Synthetic peptide sequence
<400> 22
   Gln Met Ala Lys Lys
   1               5
<210> 23
   <211> 5
   <212> PRT
   <213> Artificial Sequence
<220>
   <223> Synthetic peptide sequence
<400> 23
   Gln Tyr Lys Ser Ser
   1               5
<210> 24
   <211> 5
   <212> PRT
   <213> Artificial Sequence
<220>
   <223> Synthetic peptide sequence
<400> 24
   Arg Lys Ala Asn Asn
   1               5
<210> 25
   <211> 5
   <212> PRT
   <213> Artificial Sequence
<220>
   <223> Synthetic peptide sequence
<400> 25
   Arg Asn Asn Gln Val
   1 5
<210> 26
   <211> 5
   <212> PRT
   <213> Artificial Sequence
<220>
   <223> Synthetic peptide sequence
<400> 26
   Val Asn Ala Lys Lys
   1 5
<210> 27
   <211> 5
   <212> PRT
   <213> Artificial Sequence
<220>
   <223> Synthetic peptide sequence
<400> 27
   Val Gln Ala Lys Lys
   1 5
<210> 28
   <211> 5
   <212> PRT
   <213> Artificial Sequence
<220>
   <223> Synthetic peptide sequence
<400> 28
   Val Gln Met Phe Lys
   1 5